# EUROPEAN PATENT SPECIFICATION

(11) **EP 2 054 017 B1**
(45) Date of publication and mention of the grant of the patent: **12.10.2011**
(21) Application number: 07802601.0
(22) Date of filing: 14.08.2007
(51) Int. Cl.: A61K 8/35, A61K 8/36, A61K 8/86, A61Q 17/04, A61K 8/39

(54) **PHOTOSTABLE COSMETIC COMPOSITIONS**
FOTOSTABILE KOSMETISCHE ZUSAMMENSETZUNGEN
COMPOSITIONS COSMÉTIQUES PHOTOSTABLES

(30) Priority: 24.08.2006 IN MM13222006; 27.02.2007 EP 07003975
(43) Date of publication of application: 06.05.2009
(73) Proprietor: Unilever PLC, London EC4Y 0DY (GB); Unilever N.V., 3013 AL Rotterdam (NL)
(72) Inventor: BANDYOPADHYAY, Prasun, Mumbai 560 066 (IN); KUMAR, Gaurav, Mumbai 560 066 (IN)
(74) Representative: Acham, Nicholas Clive
(86) International application number: PCT/EP2007/058409
(87) International publication number: WO 2008/022946

(56) References cited:
- EP-A2- 0 843 996
- WO-A-00/45784
- WO-A-96/14053
- US-A- 5 985 251

## Description

The present invention relates to photostable cosmetic compositions comprising sunscreens, more particularly to cosmetic compositions comprising dibenzoylmethane sunscreens.

It is generally known that UV-A rays having wavelengths between 320 and 400 nm cause tanning of the skin, localized irritation, sun-burn and melanoma. It is also known that UV-B radiation, having wavelength between 280 nm and 320 nm also promotes tanning of the human epidermis, in addition to causing various other short and long-term damages, such as photoaging of skin, dryness, deep wrinkle formation, mottled pigmentation and the breakdown of elastic tissues and collagen. Therefore, it is desirable to protect the skin from the harmful effects of ultraviolet radiation.

Various cosmetic preparations have been reported for preventing and/or protecting the skin from harmful effects of ultraviolet radiation. Numerous organic sunscreen agents capable of absorbing harmful UV-A rays are also reported in the field of cosmetics amongst which a particularly advantageous organic sunscreen agent is dibenzoylmethane and its derivatives. This is because they exhibit high intrinsic absorption power. On the other hand p-methoxycinnamic acid and its derivatives are also used extensively as they are highly effective UV-B sunscreens. It is essential that cosmetic compositions contain both UV-A and UV-B sunscreens so as to provide protection over the entire range of UV radiation.

It is known that dibenzoylmethane and its derivatives are relatively sensitive to ultraviolet radiation and they decompose rapidly under the effect of sunlight. This decomposition is accelerated in the presence of UV-B sunscreens, especially p-methoxycinnamic acid and its derivatives. Owing to photochemical instability of dibenzoylmethane and its derivatives in the presence of UV-B sunscreens, especially p-methoxycinnamic acid and its derivatives, one cannot guarantee constant protection during prolonged exposure to the sun. This therefore warrants repeated applications at regular and frequent intervals by the user in order to maintain effective protection against UV rays.

On the other hand, it is also known that the protection afforded by cosmetic sunscreen compositions reduces over a period of time and typically by the end of 1 hour from application, the protection is almost negligible. Stabilization of dibenzoylmethane and its derivatives therefore becomes important so that the user achieves complete advantage of its efficacy and he does not have to resort to frequent applications.

Various methods have been reported for stabilization of dibenzoylmethane and its derivatives in cosmetic formulations, which include the use of stabilizers such as thickening copolymers, amphiphilic copolymers and micronized insoluble organic UV sunscreen agents.

In an alternative approach, US 5 985 251 (Roche Vitamins, 1999) describes light screening cosmetics wherein compositions comprising dibenzoylmethane derivatives and p-methoxycinnamic acid derivatives are stabilized by incorporating 0.5 to 12% by weight 3,3-diphenylacrylate derivatives or benzylidene camphor derivatives. It can be readily seen that the formulation must necessarily contain an additional UV-B sunscreen of the diphenylacrylate class to stabilize UV-A sunscreens, a large part of which ends up getting utilized for stabilizing the UV-A sunscreen, without providing protection, which is its primary role. Incorporation of the additional sunscreen would also add to the cost of the formulation.

Therefore there exists the need for cosmetic compositions comprising dibenzoylmethane or its derivatives, which are stabilized, especially in the presence of p-methoxycinnamic acid or its derivatives, wherein specialty polymers and/or additional sunscreen stabilizers are not essentially required. It is highly desirable to have cosmetic compositions, which are stabilized with ingredients that are conventionally used in cosmetics, thereby reducing the complexities of formulation and substantially reduce costs. The present inventors have surprisingly found that cosmetic compositions comprising dibenzoylmethane or its derivative and p-methoxycinnamic acid or its derivative, can be stabilized by incorporating a combination of fatty alcohol ethoxylates and polyalkyleneglycol.

It is therefore an object of the present invention to obviate at least some drawbacks of the prior art and provide photostable cosmetic compositions having sunscreens.

Another object of the present invention is to provide photostable compositions comprising dibenzoylmethane sunscreens, wherein the stabilization is brought about by using conventionally used ingredients.

### Summary of the invention

According to one aspect, the present invention relates to a photostable cosmetic composition comprising,
0.1 % to 10 % by weight dibenzoylmethane or its derivative;
0.1 % to 10 % by weight p-methoxy cinnamic acid or its derivative, wherein said composition comprises from 0.5 % to 8 % by weight C8-C18 fatty alcohol ethoxylate and 0.5 % to 8 % by weight polyalkyleneglyool, and wherein said composition is in the form of a cream or a lotion.

Preferably dibenzoylmethane or its derivative is present from 0.1 % to 5 % by weight, more preferably from 0.1% to 2% by weight of the composition.

It is also preferred that p-methoxy cinnamic acid or its derivative is present from 0.1 % to 5 % by weight, more preferably from 0.1% to 2% by weight of the composition.

According to a most preferred aspect, the dibenzoylmethane derivative is 4-tert-butyl-4'-methoxydibenzoylmethane and p-methoxynnamic acid derivative is 2-ethyl-hexyl-p-methoxycinnamate.

It is preferred that the fatty alcohol ethoxylate is present from 0.5 % to 4 % by weight of the composition.

According to a preferred aspect, the polyalkyleneglycol is present from 0.5 to 4 % by weight.

According to a preferred aspect, the molecular weight of polyethyleneglycol is between 200 to 100000 Daltons, more preferably between 200 to 10000 Daltons.

As used herein, the term "cosmetic composition" is intended to describe compositions for topical application to human skin, including leave-on and wash-off products. The term "skin" as used herein includes the skin an the face, neck, chest, back, arms, hands, legs, and scalp.

For the avoidance of doubt the word "comprising" is intended to mean including but not necessarily consisting of or composed of. In other words the listed options need not be exhaustive.

Other characteristics, aspects and advantages of the invention and the essential, preferred and optional features of the invention will emerge on reading the detailed description that follows.

### Detailed Description of the Invention

Preferred dibenzoylmethane derivative are selected from 4-tert-butyl-4'-methoxydibenzoylmethane, 2-methyldibenzoylmethane, 4-methyl-dibenzoyl-ethane, 4-isopropyldibenzoyl-methane, 4-tert-butyldibenzoylmethane, 2,4-dimethyldibenzoylmethane, 2,5-dimethyldibenzoylmethane, 4,4'-diisopropyl-dibenzoylmethane, 2-methyl-5-isopropyl-4'-methoxydibenzoylmethane, 2-methyl-5-tert-butyl-4'-methoxy-dibenzoylmethane, 2,4-dimethyl-4'-methoxydibenzoylmethane or 2,6-dimethyl-4-tert-butyl-4'-methoxy-dibenzoylmethane. The most preferred dibenzoylmethane derivative is 4-tert.-butyl-4'-methoxydibenzoylmethane.

The preferred p-methoxycinnamic acid derivative are selected from 2-ethylhexyl-p-methoxycinnamate, ammonium-p-methoxycinnamate, sodium-p-methoxycinnamate, potassium-p-methoxycinnamate, or salts of primary, secondary or tertiary amines of p-methoxycinnamic acid and more preferably it is 2-ethylhexyl-p-methoxy cinnamate.

Fatty alcohol ethoxylates (also known as ethoxylated fatty alcohols) have the general formula:

R-O-(CH2-CH2-O)n H

where R is a saturated or unsaturated, linear or branched hydrocarbon-based chain having from 10 to 24 carbon atoms, and n is an integer ranging from 8 to 50.

Fatty alcohol ethoxylates are products of the addition of ethylene oxide onto primary alcohols. The ethoxylates are produced by known methods and are basically mixtures. Depending on their production, they may have a conventional broad homolog distribution or a narrow homolog distribution. The degree of ethoxylation (EO: number of ethylene oxide units added on) represents a Gauss distribution, the maximum of the Gauss curve being referred to here as the average degree of ethoxylation "n".

Preferred are products of the addition of ethylene oxide onto caproic alcohol, caprylic alcohol, 2-ethylhexyl alcohol, capric alcohol, lauryl alcohol, isotridecyl alcohol, myristyl alcohol, cetyl alcohol, palmitoleyl alcohol, stearyl alcohol, isostearyl alcohol, oleyl alcohol, elaidyl alcohol, petroselinyl alcohol, linolyl alcohol, linolenyl alcohol, elaeostearyl alcohol, arachyl alcohol, gadoleyl alcohol, behenyl alcohol, erucyl alcohol and brassidyl alcohol and technical mixtures thereof. Representative examples of ethoxylated fatty alcohols are the addition products of ethylene oxide with lauryl alcohol, in particular those containing from 9 to 50 oxyethylenated groups (having CTFA names Laureth-9 to Laureth-50); the addition products of ethylene oxide with behenyl alcohol, in particular those containing from 9 to 50 oxyethylenated groups (having CTFA names Beheneth-9 to Beheneth-50); the addition products of ethylene oxide with cetearyl alcohol (mixture of cetyl alcohol and of stearyl alcohol) in particular those containing from 9 to 30 oxyethylenated groups (having CTFA names Ceteareth-9 to Ceteareth-30); the addition products of ethylene oxide with cetyl alcohol, in particular those containing from 9 to 30 oxyethylenated groups (having CTFA names Ceteth-9 to Ceteth-30); the addition products of ethylene oxide with stearyl alcohol, in particular those containing from 9 to 30 oxyethylenated groups (having CTFA names Steareth-9 to Steareth-30; the addition products of ethylene oxide with isostearyl alcohol, in particular those containing from 9 to 50 oxyethylenated groups (having CTFA names Isosteareth-9 to Isosteareth-50); and mixtures thereof.

The more preferred alcohol ethoxylates are addition products of ethylene oxide onto fatty alcohol having 8 to 18 carbon atoms, i.e. C8-C18 fatty alcohol ethoxylates, and in a highly preferred aspect; it is lauryl alcohol, which has 12 carbon atoms. It is marketed under the name Brij® 35 (laureth-35, lauryl alcohol with 35 EO units). Several grades of Brij are available, depending upon the degree of ethoxylation.

A suitable polyalkyleneglycol is selected from polyethyleneglycol, polypropyleneglycol or polybutyleneglycol and preferably it is polyethyleneglycol.

The present composition can include any cosmetic vehicle/carrier known in the art. Suitable vehicles include, but are not limited to, one or more of the following: vegetable oils; esters such as octyl palmitate, isopropyl myristate and isopropyl palmitate; ethers such as dicapryl ether and dimethyl isosorbide; alcohols such as ethanol and isopropanol; fatty alcohols such as cetyl alcohol, stearyl alcohol and behenyl alcohol; isoparaffins such as isooctane, isododecane and isohexadecane; silicone oils such as dimethicones, cyclic silicones, and polysiloxanes; hydrocarbon oils such as mineral oil, petrolatum, isoeicosane and polyisobutene; polyols such as propylene glycol, ethoxydiglycol, glycerin, butylene glycol, pentylene glycol and hexylene glycol; as well as water, or any combinations of the above. Fatty acids having from 10 to 30 carbon atoms may also be included as cosmetically acceptable carriers for compositions of this invention. Illustrative of this category are pelargonic, lauric, myristic, palmitic, stearic, isostearic, hydroxystearic, oleic, linoleic, ricinoleic, arachidic, behenic and erucic acids.

Humectants of the polyhydric alcohol-type may also be employed as cosmetically acceptable carriers in compositions of this invention. The humectant aids in increasing the effectiveness of the emollient, reduces skin dryness and improves skin feel. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, 1,2, 6-hexanetriol, ethoxylated glycerol, propoxylated glycerol and mixtures thereof. The amount of humectant may range anywhere from 0.5% to 30%, preferably between 1% and 15% by weight of the composition.

The amount of cosmetically acceptable vehicle in the present composition will vary considerably based upon product form, but typically will range from about 20 wt % to about 70 wt % and preferably from about 20 wt % to about 40 wt %, based upon the total weight of the composition. The cosmetically acceptable vehicle acts as a dilutant, dispersant or carrier for the in the composition, so as to facilitate the distribution of the sunscreens when the composition is applied to the skin.

The present composition, when in emulsion form, could optionally have one or more additional emulsifiers, without deviating from the scope of the invention, which are preferably selected from sorbitan esters dimethicone copolyols; polyglyceryl-3-diisostearate; such as sorbitan monooleate and sorbitan monostearate; glycerol esters such as glycerol monostearate and glycerol monooleate; polyoxyethylene phenols such as polyoxyethylene octyl phenol and polyoxyethylene nonyl phenol; polyoxyethylene ethers such as polyoxyethylene cetyl ether and polyoxyethylene stearyl ether; polyoxyethylene glycol esters; polyoxyethylene sorbitan esters; dimethicone copolyols; polyglyceryl-3-diisostearate; or any combinations thereof. An oil or oily material may be present, together with an emulsifier to provide either a water-in-oil emulsion or an oil-in-water emulsion, depending largely on the average hydrophilic-lipophilic balance (HLB) of the emulsifier employed. Preferred anionic surfactants include soap, alkyl ether sulfate and sulfonates, alkyl sulfates and sulfonates, alkylbenzene sulfonates, alkyl and dialkyl sulfosuccinates, C9-C20 acyl isethionates, acyl glutamates,C8-C20 alkyl ether phosphates and combinations thereof. Typically, the additional emulsifier could be present from 1 wt % to about 12 wt %, based upon the total weight of the composition. Water when present will be in amounts which could range from 5% to 75%, preferably from 20% to 70%, optimally between 40% and 70% by weight of said composition.

Besides water, relatively volatile solvents may also serve as carriers within compositions of the present invention. Most preferred are monohydric C1-C3 alkanols. These include ethyl alcohol and isopropyl alcohol.

Preferred cream bases are, for example, beeswax, cetyl alcohol, stearic acid, glycerine, propylene glycol, propylene glycol monostearate, polyoxyethylene cetyl ether and the like. Preferred lotion bases include, for example, oleyl alcohol, ethanol, propylene glycol, glycerine, lauryl ether, sorbitan monolaurate and the like.

When the composition of the present invention is in the form of film-forming skin packs or masks it could comprise film formers known in the art. These include acrylate copolymers, acrylates C12-22 alkyl methacrylate copolymer, acrylate/octylacrylamide copolymers, acrylate/VA copolymer, amodimethicone, AMP/acrylate copolymers, behenyl beeswax, behenyl/isostearyl, beeswax, butylated PVP, butyl ester of PVM/MA copolymers, calcium/sodium PVM/MA copolymers, dimethicone, dimethicone copolyol, dimethicone/mercaptopropyl methicone copolymer, dimethicone propylethylenediamine behenate, dimethicolnol ethylcellulose, ethylene/acrylic acid copolymer, ethylene/MA copolymer, ethylene/VA copolymer, fluoro C2-8 alkyldimethicone, hexanediol beeswax, C30-38 olefin/isopropyl maleate/MA copolymer, hydrogenated styrene/butadiene copolymer, hydroxyethyl ethylcellulose, isobutylene/MA copolymer, laurylmethicone copolyol, methyl methacrylate crosspolymer, methylacryloyl ethyl betaine/acrylates copolymer, microcrystalline wax, nitrocellulose, octadecene/MA copolymer, octadecene/maleic anhydride copolymer, octylacrylamide/acrylate/butylaminoethyl methacrylate copolymer, oxidized polyethylene, perfluoropolymethylisopropyl ether, polyacrylic acid, polyethylene, polymethyl methacrylate, polypropylene, polyquaternium-10, polyquaternium-11, polyquaternium-28, polyquaternium-4, PVM/MA decadiene crosspolymer, PVM/MA copolymer, PVP, PVP/decene copolymer, PVP/eicosene copolymer, PVP/hexadecene copolymer, PVP/MA copolymer, PVP/VA copolymer, silica, silica dimethyl silylate, sodium acrylate/vinyl alcohol copolymer, stearoxy dimethicone, stearoxytrimethylsilane, stearyl alcohol, stearylvinyl ether/MA copolymer, styrene/DVB copolymer, styrene/MA copolymer, tetramethyl tetraphenyl trisiloxane, tricontanyl trimethyl pentaphenyl trisiloxane, trimethylsiloxysilicate, VA/crotonates copolymer, VA/crotonates/vinyl proprionate copolymer, VA/butyl maleate/isobornyl acrylate copolymer, vinyl caprolactam/PVP/dimethylaminoethyl methacrylate copolymer, and vinyldimethicone.

The film former is preferably present in an amount from about 0.5 wt % to about 5 wt %, and more preferably from about 1 wt % to about 5 wt %, based upon the total weight of the composition. More preferably, the film former is present in an amount about 3 wt % of the total weight of the composition.

Optionally, the present composition may include one or more ingredients selected from chelating agents, botanical extracts, colorants, depigmenting agents, emollients, exfollients, fragrances, humectants, moisturizers, preservatives, skin protectants, skin penetration enhancers, stabilizers, thickeners, viscosity modifiers, vitamins, anti-aging, wrinkle- reducing, skin whitening, anti-acne, and sebum reduction agents or any combinations thereof. Examples of these include alpha-hydroxy acids and esters, beta-hydroxy acids and ester, polyhydroxy acids and esters, kojic acid and esters, ferulic acid and ferulate derivatives, vanillic acid and esters, dioic acids (such as sebacid and azoleic acids) and esters, retinol, retinal, retinyl esters, hydroquinone, t-butyl hydroquinone, mulberry extract, licorice extract, and resorcinol derivatives such as 4-substituted resorcinol derivatives, as well as additional sunscreens such UV diffusing agents, typical of which is finely divided titanium oxide and zinc oxide which generally arebetween 5 nm and 100 nm and preferably between 10 and 50 nm) of coated or uncoated metal oxides, for instance nanopigments of titanium oxide (amorphous or crystallized in rutile and/or anatase form), of iron oxide, of zinc oxide, of zirconium oxide or of cerium oxide, which are all photoprotective agents that are well-known per se, acting by physically blocking out (reflection and/or scattering) UV radiation. Standard coating agents are, moreover, alumina and/or aluminum stearate.. The compositions according to the invention may also contain agents for artificially tanning and/or browning the skin (self-tanning agents), for instance dihydroxy-acetone (DHA).

Thickeners may also be utilized as part of the cosmetically acceptable carrier of compositions according to the present invention, without deviating from the scope of the present invention. Typical thickeners include crosslinked acrylates (e. g. Carbopol 982), hydrophobically-modified acrylates (e. g. Carbopol 1382), cellulosic derivatives and natural gums. Among useful cellulosic derivatives are sodium carboxymethylcellulose, hydroxypropyl methylcellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Amounts of the thickener may range from 0. 0001% to.5%, usually from 0. 001% to 1%, optimally from 0.01 % to 0.5% by weight.

The inventive cosmetic composition could also optionally contain lathering surfactant. By "lathering surfactant" is meant a surfactant which, when combined with water and mechanically agitated, generates a foam or lather. Preferably, the lathering surfactant should be mild, meaning that it must provide sufficient cleansing or detergent benefits but not overly dry the skin, and yet meet the lathering criteria described above. The cosmetic compositions of the present invention may contain a lathering surfactant in a concentration of about 0.01% to about 50%. This is typically needed, in wash-off products, such as face-washes.

The cosmetic compositions according to the invention may also contain, besides the essential elements, one or more additional sunscreens that are different from the preceding sunscreens, which are water-soluble, liposoluble or insoluble in the cosmetic solvents commonly used. These screening agents may be suitably chosen from salicylic derivatives, benzylidenecamphor derivatives, triazine derivatives, benzophenone derivatives, [β], [β]'-diphenylacrylate derivatives, phenyl-benzimidazole derivatives, anthranilic derivatives, imidazoline derivatives, methylenebis(hydroxyphenyl-benzotriazole) derivatives, p-aminobenzoic acid derivatives, and screening hydrocarbon-based polymers and screening silicones derivatives.

The preferred organic UV-screening agents are chosen from the following compounds: Ethylhexyl salicylate, Octocrylene, Phenylbenzimidazolesulphonic acid, Terephthalylidenedicamphorsulphonic acid, Benzophenone-3, Benzophenone-4, Benzophenone-5, 4-Methylbenzylidene, Benzimidazilate, Anisotriazine, 2,4,6-Tris(diisobutyl 4'-aminobenzalmalonate)-s-triazine, Ethylhexyltriazone, Diethylhexylbutamidotriazone, Methylenebis(benzotriazolyl)tetramethylbutyl-phenol, Drometrizole trisiloxane, and mixtures thereof.

Specific preparations of the cosmetics to which the present invention is applicable include creams and lotions. The emulsion could be, for example, anhydrous, water-in-oil, oil-in-water, water-in-silicone, or multiple emulsions. In case of protection of the hairs, the suitable formulations are shampoos, conditioners, lotions, emulsions, dispersions, lacquers, and the like.

The cosmetic composition of the invention can be formulated as a lotion having a viscosity of from 4,000 to 10,000mPas, a fluid cream having a viscosity of from 10,000 to 20,000 mPas or a cream having a viscosity of from 20,000 to 100,000 mPas or above, all measured at 25 °C.

The composition according to the invention is intended primarily as a personal care product for topical application to human skin, as well as to protect exposed skin from the harmful effects of excessive exposure to sunlight.

In use, a small quantity of the composition, for example about 0.1 ml to about 5 ml, is applied to exposed areas of the skin, from a suitable container or applicator and, if necessary, it is then spread over and/or robbed into the skin using the hand or fingers or a suitable device.

In order to further illustrate the present invention and the advantages thereof, the following specific examples are given, it being understood that same are intended only as illustrative and in no way limitative. In said examples to follow, all parts and percentages are given by weight, unless otherwise indicated.

### Examples

### Example 1

Demonstration of stabilization of dibenzoylmethane derivative using combination of fatty alcohol ethoxylate and polyethyleneglycol, according to the invention.

Various cosmetic cream compositions were prepared as per formulation details given in table 1 below.

**Table 1**

| Ingredients % wt | Control | Form 1 | Form 2 | Form 3 | Form 4 | Form 5 |
|---|---|---|---|---|---|---|
| Stearic acid | 18 | 18 | 18 | 18 | 18 | 18 |
| Potassium hydroxide | 0.67 | 0.67 | 0.67 | 0.67 | 0.67 | 0.67 |
| Cetyl alcohol | 0.53 | 0.53 | 0.53 | 0.53 | 0.53 | 0.53 |
| Isopropyl myristate | 0.75 | 0.75 | 0.75 | 0.75 | 0.75 | 0.75 |
| Dimethicone 200 | 0.5 | 0.5 | 0.5 | 0.5 | 0.5 | 0.5 |
| Niacinamide | 1 | 1 | 1 | 1 | 1 | 1 |
| Parsol 1789 | 0.8 | 0.8 | 0.8 | 0.8 | 0.8 | 0.8 |
| Parsol MCX | 0.75 | 0.75 | 0.75 | 0.75 | 0.75 | 0.75 |
| Brij-35 | - | - | 4 | 4 | - | - |
| Tween 80 | - | - | - | - | 4 | - |
| Span 80 | - | - | - | - | | 4 |
| Polyethylene glycol 200 | - | 4 | - | 4 | 4 | 4 |
| Water and other minors upto | 100 | 100 | 100 | 100 | 100 | 100 |

| | | | | | | |
|---|---|---|---|---|---|---|
| Tween 80 Polyoxythylene sorbitan monooleate (Uniquema) Span 80 Sorbitan monooleate (Uniquema) Brij-35 Polyoxyethylene lauryl ether (Uniquema) Parsol 1789 4-tert.-butyl-4'-methoxydibenzoylmethane (Merck) Parsol MCX 2-ethylhexyl methoxycinnamate (Merck) | | | | | | |

### Processing

1. Weighed quantities of water, potassium hydroxide, EDTA, p-casitose, and glycerin were taken in a 100ml beaker (vessel A).
2. Weighed quantities of isopropyl myristate, diemthicone 200, Parsol 1789, Parsol MCX, propylparaben, methylparaben, cetyl alcohol, and phenoxyethanol were taken in another beaker. (vessel B)
3. Stearic acid was melted by heating to 70°C in a separate vessel.
4. Contents of vessel B were melted at 70°C.
5. Vessel B was heated to 75°C under continuous homogenization at low shear (500-700 rpm).
6. After attaining 70°C, molten stearic acid was added to vessel A, slowly and under continuous stirring.
7. After 1-2 min of stirring Brij 35/Tween 80/Span 80 was added, followed by the addition of PEG-200 in above rotating mixture, followed by the addition of TiO2.
8. Molten mass of vessel B was added to it.
9. Shear rate of homogenization was increased to 1000-1200 rpm and was continued for 5 min.
10. The hot mixture was thereafter allowed to cool at room temperature with continuous stirring at low speed (500-700rpm).
11. At 60°C, niacinamide was added.
12. Mixture was allowed to cool to room temperature.

### Test Method

The following test method was used for determining the stability of dibenzoylmethane sunscreens in the compositions of the present invention as well as for all the comparative examples described below.
1. A clean glass slide was taken and its weight was recorded (A).
2. About 10mg of cream was applied and spread on about 2 cm².
3. The weight of slide with cream was recorded as (B).
4. Subtraction of B from A gave the weight of cream applied (C).
5. The above-mentioned process was repeated six times for each test formulation.
6. These glass slides were exposed to sun simultaneously for various time intervals: 0 min, 15 min, 30 min and 60 min respectively.
7. After exposure to sunlight the creams were extracted in methanol and the volume was made up to 25ml.
8. The UV-absorbance of each of the samples was recorded on a UV spectrophotometer.
9. Absorbance per unit weight of the sample was calculated by dividing the absorbance at λ max (i.e. 357nm, which is λ max of dibenzoylmethanes) by weight of the cream (C).
10. The percentage absorbance remaining which is an indicator of the stability of the sample was calculated as follows:

Percentage absorbance remaining = [Aₙ / A₀ X 100] where A₀ is "absorbance per unit weight" of 0 min sample, and Aₙ is "absorbance per unit weight" of nth min sample. The results of the experiment conducted in example 1 are summarized in table 2 below. It is to be noted that the intensity of sunlight was found to vary from 20-40 mW/Cm² at the time of exposure of the slides to sun and the experiments were conducted on different days. Therefore, the absolute values of % absorbance for the same/similar experiments conducted over the entire period, e.g. experiments using control samples are different across the tables.

**Table 2**

| Sunlight Exposure time/min | % Absorbance of Parsol 1789 remaining after exposure, when measured by the procedure given above. | | | |
|---|---|---|---|---|
| | Control | Form 1 | Form 2 | Form 3 |
| 0 (T₀) | 100 | 100 | 100 | 100 |
| 15 (T₁₅) | 79 | 91 | 78. | 97 |
| 30(T₃₀) | 68 | 91 | 69 | 93 |
| 45 (T₄₅) | 60 | 80 | 60 | 91 |
| 60 (T₆₀) | 42 | 69 | 57 | 88 |

The above table indicates that in the formulation containing both PEG 200 and Brij-35, a significantly higher activity of dibenzoylmethane sunscreen is available after 1 hour of application, as evident from the absorbance value.

### Example 2:

To study the effect of combination of polyethyleneglycol with other surfactants on the stability of dibenzoylmethane sunscreen and its comparison with the combination of polyethyleneglycol with Brij 35 according to the invention.

The results are summarized in table 3 below.

**Table 3**

| Sunlight Exposure time/min | % Absorbance of Parsol 1789 remaining after exposure, when measured by the procedure given above. | | | |
|---|---|---|---|---|
| | Control | Form 4 | Form 5 | Form 3 |
| 0 (T₀) | 100 | 100 | 100 | 100 |
| 15 (T₁₅) | 66 | 73 | 96 | 97 |
| 30(T₃₀) | 66 | 71 | 78 | 94 |
| 45 (T₄₅) | 47 | 63 | 78 | 85 |
| 60 (T₆₀) | 40 | 64 | 75 | 83 |

Thus it can be readily seen that a combination of polyethylene glycol and fatty alcohol ethoxylate gives better stability as compared to combination of polyethylene glycol and other surfactants/emulsifiers.

### Example 3

The present inventors have also determined the effect of varying the molecular weight of polyethyleneglycol on the stability of dibenzoylmethane. The results of this experiment are summarized in table 4 below.

**Table 4**

| Sunlight Exposure time/min | % Absorbance of Parsol 1789 remaining after exposure, when measured by the procedure given above. | | | | |
|---|---|---|---|---|---|
| | Control | Form 3 | Form 3 with 4% PEG 6000 instead of 4% PEG 200 | Form 3 with 4% PEG 20000 instead of 4% PEG 200 | Form 3 with 4% PEG 100000 instead of 4% PEG 200 |
| 0 (T₀) | 100 | 100 | 100 | 100 | 100 |
| 15 (T₁₅) | 74 | 86 | 91 | 86 | 96 |
| 30(T₃₀) | 57 | 81 | 87 | 80 | 81 |
| 45 (T₄₅) | 50 | 78 | 78 | 68 | 76 |
| 60 (T₆₀) | 47 | 72 | 71 | 70 | 72 |

The above table indicates that with various molecular weights of polyethylene glycol, greater than 70% of the dibenzoylmethane derivative remains available, even after 1 hour from application. This demonstrates that any molecular weight of PEG from 200 to 100000 can be used, without departing from the scope of the invention.

### Example 4

In yet another set of experiments, the effect of 2 grades of fatty alcohol ethoxylates (Brij) on the stability of dibenzoylmethane sunscreen was studied in cosmetic creams, the results of which are summarized in table 5 below.

**Table 5**

| Sunlight Exposure time/min | % Absorbance of Parsol 1789 remaining after exposure, when measured by the procedure given below. | | | |
|---|---|---|---|---|
| | Control | Form 1 | Form 3 | Form-3 with 4% Brij 56 instead of 4% Brij 35. |
| 0 (T₀) | 100 | 100 | 100 | 98 |
| 15 (T₁₅) | 81 | 81 | 99 | 84 |
| 30(T₃₀) | 70 | 69 | 95 | 78 |
| 45 (T₄₅) | 45 | 68 | 84 | 74 |
| 60 (T₆₀) | 43 | 59 | 83 | 74 |

| | | | | |
|---|---|---|---|---|
| (Note: Brij 56 is polyethylene glycol hexadecyl ether or polyoxyethylene 10 cetyl ether(CAS number 9004-95-9) (Uniquema or Sigma-Aldrich)) | | | | |

Thus, it can be readily seen that different grades of fatty alcohol ethoxylates provide a high degree of stability to the dibenzoylmethane sunscreen in the composition.

### Example 5

The stability of dibenzoylmethane derivative was also studied in the case of cosmetic lotions, to test the invention in a different carrier/vehicle. The details of the formulation are present in table 6 below. The processing details are also given below. The Results are summarized in the table 7 below.

**Table 6**

| Ingredient | Percentage | | | |
|---|---|---|---|---|
| | Control lotion | Lotion A | Lotion B | Lotion C |
| Part A | | | | |
| Water | 84 | 84 | 84 | 84 |
| | | | | |
| Part B | | | | |
| | | | | |
| Brij 35 | - | 4.00 | - | 4.00 |
| | | | | |
| Peg-200 | - | - | 4.00 | 4.00 |
| | | | | |
| Part C | | | | |
| Titanium Dioxide | 1.00 | 1.00 | 1.00 | 1.00 |
| | | | | |
| Part D | | | | |
| Isopropyl Myristate | 0.75 | 0.75 | 3.75 | 0.75 |
| Dimethicone DC 200 | 0.50 | 0.50 | 0.50 | 0.50 |
| Stearic acid | 2.00 | 2.00 | 2.00 | 2.00 |
| Cetyl alcohol | 0.53 | 0.53 | 0.53 | 0.53 |
| Parsol 1789 | 0.80 | 0.80 | 0.80 | 3.80 |
| Parsol MCX | 0.75 | 0.75 | 0.75 | 0.75 |
| Part E | | | | |
| Triethanolamine (99%) | 0.50 | 0.50 | 0.50 | 0.50 |
| Total with other minors | 100 | 100 | 100 | 100 |

### Processing

1. Disodium EDTA was added to the water and was mixed until dissolution.
2. Carbopol Ultrez (TM) was dispersed in the water and was mixed at low speed.
3. The ingredients of Part B were added to the water.
4. The ingredients of Part C were added to the water and mixed after moderate heating. The TiO2 was mixed until it dispersed.
5. The combined Parts A, B and C were heated to 65°C.
6. The ingredients of Part D were heated to 65°C and mixed until all the solids dissolved.
7. This part D was then added to combined Parts A, B and C. While the temperature was at 65°C, the Part E ingredients were added.
8. The emulsion was mixed under moderate agitation until the temperature reached 40°C. It was later cooled to room temperature and was used as such for analysis.

**Table 7**

| Sunlight Exposure time/min | % Absorbance of Parsol 1789 remaining after exposure, when measured by the procedure given above. | | | |
|---|---|---|---|---|
| | Control lotion | Lotion A | Lotion B | Lotion C |
| 0 (T₀) | 100 | 100 | 100 | 100 |
| 15 (T₁₅) | 81 | 96 | 81 | 86 |
| 30(T₃₀) | 70 | 91 | 69 | 78 |
| 45 (T₄₅) | 46 | 70 | 68 | 75 |
| 60 (T₆₀) | 43 | 68 | 59 | 76 |

Thus it can be readily seen that the inventive combination of polyethylene glycol and fatty alcohol ethoxylate stabilizes the dibenzoylmethane derivative, even in a lotion based cosmetic composition.

Thus it can be seen from the foregoing description and examples that the invention provides for a composition comprising stable sunscreens. The invention also provides for compositions comprising stabilized dibenzoylmethane sunscreens, wherein the stabilization is brought about by using ingredients, which are conventionally used in cosmetic compositions.

While the invention has been described in terms of various specific and preferred embodiments, the skilled artisan will appreciate that various modifications, substitutions, omissions, and changes may be made without departing from the spirit thereof.

## Claims

1. A photostable cosmetic composition comprising,
a) 0.1 % to 10 % by weight dibenzoylmethane or its derivative;
b) 0.1 % to 10 % by weight p-methoxycinnamic acid or its derivative,
wherein said composition comprises from 0.5 % to 8 % by weight C8-C18 fatty alcohol ethoxylate and 0.5 % to 8 % by weight polyalkyleneglycol, and wherein said composition is in the form of a cream or a lotion.

2. A photostable cosmetic composition as claimed in claim 1, wherein said dibenzoylmethane or its derivative is present from 0.1 % to 5 % by weight.

3. A photostable cosmetic composition as claimed in claim 1 or claim 2, wherein said p-methoxycinnamic acid or its derivative is present from 0.1 % to 5 % by weight.

4. A photostable cosmetic composition as claimed in any preceding claim, wherein said p-methoxycinnamic acid or its derivative is present from 0.1 % to 2 % by weight.

5. A photostable cosmetic composition as claimed in any preceding claim, wherein said dibenzoylmethane derivative is selected from 4-tert-butyl-4'-methoxydibenzoylmethane, 2-methyldibenzoylmethane, 4-methyl-dibenzoylethane, 4-isopropyldibenzoyl-methane, 4-tert-butyldibenzoylmethane, 2,4-dimetthyldibenzoylmethane, 2,5-dimethyldibenzoylmethane, 4,4'-diisopropyldibenzoylmethane, 2-methyl-5-isopropyl-4' -methoxydibenzoylmethane, 2-methyl-5-tert-butyl-4' -methoxy-dibenzoylmethane, 2,4-dimethyl-4'-methoxydibenzoylmethane or 2,6-dimethyl-4-tert-butyl-4'-methoxydibenzoylmethane.

6. A photostable cosmetic composition as claimed in 5, wherein said dibenzoylmethane derivative is 4-tert. -butyl-4'-methoxydibenzoylmethane.

7. A photostable cosmetic composition as claimed in any preceding claim, wherein said p-methoxycinnamic acid derivative is selected from 2-ethylhexyl-4-methoxycinnamate, ammonium p-methoxycinnamate, sodium p-methoxycinnamate, potassium p-methoxycinnamate, or salts of primary, secondary or tertiary amines of p-methoxycinnamic acid.

8. A photostable cosmetic composition as claimed in claim 7, wherein said p-methoxycinnamic acid derivative is 2-ethylhexyl-p-methoxycinnamate.

9. A photostable cosmetic composition as claimed in any preceding claim, wherein said C8-C18 fatty alcohol ethoxylate is present from 0.5 % to 4 % by weight.

10. A photostable cosmetic composition as claimed in any preceding claim, wherein said polyalkyleneglycol is present from 0.5 % to 4 % by weight of said composition.

11. A photostable cosmetic composition as claimed in any preceding claim, wherein said polyalkyleneglycol is selected from polyethyleneglycol, polypropyleneglycol or polybutyleneglycol.

12. A photostable cosmetic composition as claimed in claim 12, wherein molecular weight of said polyethyleneglycol is from 200 to 100000 Daltons.

13. A photostable cosmetic composition as claimed in claim 13, wherein said molecular weight is from 200 to 10000 Daltons.

## Patentansprüche

1. Fotostabile kosmetische Zusammensetzung, umfassend
a) 0,1 bis 10 Gewichts-% an Dibenzoylmethan oder seinem Derivat;
b) 0,1 bis 10 Gewichts-% an p-Methoxyzimtsäure oder ihrem Derivat,
wobei die Zusammensetzung 0,5 bis 8 Gewichts-% C8-C18-Fettalkoholethoxylat und 0,5 bis 8 Gewichts-% Polyalkylenglykol umfasst und wobei die Zusammensetzung in der Form einer Creme oder einer Lotion ist.

2. Fotostabile kosmetische Zusammensetzung, wie sie in Anspruch 1 beansprucht ist, wobei das Dibenzoylmethan oder sein Derivat mit 0,1 bis 5 Gewichts-% vorliegt.

3. Fotostabile kosmetische Zusammensetzung, wie sie in Anspruch 1 oder Anspruch 2 beansprucht ist, wobei die p-Methoxyzimtsäure oder ihr Derivat mit 0,1 bis 5 Gewichts-% vorliegt.

4. Fotostabile kosmetische Zusammensetzung, wie sie in einem vorangehenden Anspruch beansprucht ist, wobei die p-Methoxyzimtsäure oder ihr Derivat mit 0,1 bis 2 Gewichts-% vorliegt.

5. Fotostabile kosmetische Zusammensetzung, wie sie in einem vorangehenden Anspruch beansprucht ist, wobei das Dibenzoylmethanderivat aus 4-tert-Butyl-4'-methoxydibenzoylmethan, 2-Methyldibenzoylmethan, 4-Methyldibenzoylethan, 4-Isopropyldibenzoylmethan, 4-tert-Butyldibenzoylmethan, 2,4-Dimethyldibenzoylmethan, 2,5-Dimethyldibenzoylmethan, 4,4'-Diisopropyldibenzoylmethan, 2-Methyl5-isopropyl-4'-methoxydibenzoylmethan, 2-Methyl-5-tert-butyl-4'-methoxydibenzoylmethan, 2,4-Dimethyl-4'-methoxydibenzoylmethan oder 2,6-Dimethyl-4-tert-butyl-4'-methoxydibenzoylmethan ausgewählt ist.

6. Fotostabile kosmetische Zusammensetzung, wie sie in Anspruch 5 beansprucht ist, wobei das Dibenzoylmethanderivat 4-tert-Butyl-4'-methoxydibenzoylmethan ist.

7. Fotostabile kosmetische Zusammensetzung, wie sie in einem vorangehenden Anspruch beansprucht wird, wobei das p-Methoxyzimtsäurederivat aus 2-Ethylhexyl-4-methoxycinnamat, Ammonium-p-methoxycinnamat, Natrium-p-methoxycinnamat, Kalium-p-methoxycinnamat und Salzen von primären, sekundären oder tertiären Aminen von p-Methoxyzimtsäure ausgewählt ist.

8. Fotostabile kosmetische Zusammensetzung, wie sie in Anspruch 7 beansprucht ist, wobei das p-Methoxyzimtsäurederivat 2-Ethylhexyl-p-methoxycinnamat ist.

9. Fotostabile kosmetische Zusammensetzung, wie sie in einem vorangehenden Anspruch beansprucht ist, wobei das C8-C18-Fettalkoholethoxylat mit 0,5 bis 4 Gewichts-% vorliegt.

10. Fotostabile kosmetische Zusammensetzung, wie sie in einem vorangehenden Anspruch beansprucht ist, wobei das Polyalkylenglykol mit 0,5 bis 4 Gewichts-% der Zusammensetzung vorliegt.

11. Fotostabile kosmetische Zusammensetzung, wie sie in einem vorangehenden Anspruch beansprucht ist, wobei das Polyalkylenglykol aus Polyethylenglykol, Polypropylenglykol und Polybutylenglykol ausgewählt ist.

12. Fotostabile kosmetische Zusammensetzung, wie sie in Anspruch 12 beansprucht ist, wobei das Molekulargewicht des Polyethylenglykols 200 bis 100000 Dalton ist.

13. Fotostabile kosmetische Zusammensetzung, wie sie in Anspruch 13 beansprucht ist, wobei das Molekulargewicht von 200 bis 10000 Dalton ist.

## Revendications

1. Composition cosmétique photostable comprenant,
a) 0,1 à 10 % en poids de dibenzoylméthane ou son dérivé ;
b) 0,1 à 10 % en poids d'acide p-méthoxycinnamique ou son dérivé ;
dans laquelle ladite composition comprend de 0,5 à 8 % en poids d'éthoxylate d'alcool gras C₈-C₁₈ et de 0,5 à 8 % en poids de polyalkylène glycol, et dans laquelle ladite composition est sous la forme d'une crème ou d'une lotion.

2. Composition cosmétique photostable selon la revendication 1, dans laquelle ledit dibenzoylméthane ou son dérivé est présent de 0,1 à 5 % en poids.

3. Composition cosmétique photostable selon la revendication 1 ou la revendication 2, dans laquelle ledit acide p-méthoxycinnamique ou son dérivé est présent de 0,1 à 5 % en poids.

4. Composition cosmétique photostable selon l'une quelconque des revendications précédentes, dans laquelle ledit acide p-méthoxycinnamique ou son dérivé est présent de 0,1 à 2 % en poids.

5. Composition cosmétique photostable selon l'une quelconque des revendications précédentes, dans laquelle ledit dérivé de dibenzoylméthane est choisi parmi le 4-tert-butyl-4'-méthoxydibenzoylméthane, le 2-méthyldibenzoylméthane, le 4-méthyl-dibenzoyléthane, le 4-isopropyldibenzoyl-méthane, le 4-tert-butyldibenzoylméthane, le 2,4-diméthyldibenzoylméthane, le 2,5-diméthyldibenzoylméthane, le 4,4'-diisopropyldibenzoylméthane, le 2-méthyl-5-isopropyl-4'-méthoxydibenzoylméthane, le 2-méthyl-5-tert-butyl-4'-méthoxy-dibenzoylméthane, le 2,4-diméthyl-4'-méthoxydibenzoylméthane ou le 2,6-diméthyl-4-tert-butyl-4'-méthoxydibenzoylméthane.

6. Composition cosmétique photostable selon la revendication 5, dans laquelle ledit dérivé de dibenzoylméthane est le 4-tert-butyl-4'-méthoxydibenzoylméthane.

7. Composition cosmétique photostable selon l'une quelconque des revendications précédentes, dans laquelle ledit dérivé d'acide p-méthoxycinnamique est choisi parmi le 2-éthylhexyl-4-méthoxycinnamate, le p-méthoxycinnamate d'ammonium, le p-méthoxycinnamate de sodium, le p-méthoxycinnamate de potassium, ou les sels d'amines primaires, secondaires ou tertiaires de l'acide p-méthoxycinnamique.

8. Composition cosmétique photostable selon la revendication 7, dans laquelle ledit dérivé d'acide p-méthoxycinnamique est le 2-éthylhexyl-p-méthoxycinnamate.

9. Composition cosmétique photostable selon l'une quelconque des revendications précédentes, dans laquelle ledit éthoxylate d'alcool gras C₈-C₁₈ est présent de 0,5 à 4 % en poids.

10. Composition cosmétique photostable selon l'une quelconque des revendications précédentes, dans laquelle ledit polyalkylène glycol est présent de 0,5 à 4 % en poids de ladite composition.

11. Composition cosmétique photostable selon l'une quelconque des revendications précédentes, dans laquelle ledit polyalkylène glycol est choisi parmi le polyéthylène glycol, le polypropylène glycol ou le polybutylène glycol.

12. Composition cosmétique photostable selon la revendication 12, dans laquelle le poids moléculaire dudit polyéthylène glycol est de 200 à 100 000 Daltons.

13. Composition cosmétique photostable selon la revendication 13, dans laquelle ledit poids moléculaire est de 200 à 10 000 Daltons.
